# EUROPÄISCHE PATENTANMELDUNG

(11) **EP 4 570 784 A1**
(43) Veröffentlichungstag der Anmeldung: **18.06.2025**
(21) Anmeldenummer: 23216712.2
(22) Anmeldetag: 14.12.2023
(51) Int. Cl.: C07C 263/10, C07C 265/14, B01D 1/00

(54) **VERFAHREN UND VORRICHTUNG ZUR HERSTELLUNG VON ISOCYANATEN**

(71) Anmelder: Covestro Deutschland AG, 51373 Leverkusen (DE)
(72) Erfinder: Merkel, Michael, 40223 Düsseldorf (DE); Borrmann, Ruediger, 51063 Köln (DE); Loddenkemper, Tim, 41542 Dormagen (DE); Schade, Dieter, 51467 Bergisch Gladbach (DE)
(74) Vertreter: Levpat

(57) **Zusammenfassung**

Die Erfindung betrifft ein Verfahren zur Herstellung von Isocyanaten durch Phosgenierung von Aminen mit Phosgen, eine Verwendung und eine Phosgenierungsanlage zur Durchführung des Verfahrens.

## Beschreibung

Die Erfindung betrifft ein Verfahren zur Herstellung von Isocyanaten durch Phosgenierung von Aminen in einer Phosgenierungsanlage, bei dem Probleme infolge der Bildung von festen Amin-Ablagerungen in aminführenden Anlagenteilen, insbesondere während geplanter oder ungeplanter Anlagenstillstände vermindert oder gänzlich vermieden werden. Darüber hinaus betrifft die Erfindung eine Verwendung und eine Phosgenierungsanlage zur Durchführung des erfindungsgemäßen Verfahrens.

Die großtechnische Herstellung von Isocyanaten durch Umsetzung der korrespondierenden Amine mit Phosgen ist seit längerem aus dem Stand der Technik bekannt, wobei die Reaktion in der Gas- oder Flüssigphase kontinuierlich oder diskontinuierlich stattfinden kann. Von besonderer Bedeutung sind Di- oder Polyisocyanate, die sich zur Herstellung von Polymeren, wie beispielswiese Polyurethanen, Polythiourethanen, Polyisocyanuraten oder Polyharnstoffen eignen. Es kommen dabei sowohl aromatische Isocyanate wie beispielsweise Methylendiphenyldiisocyanat bzw. dessen höhere Homologe, Toluylendiisocyanat als auch aliphatische Isocyanate wie beispielsweise Hexamethylendiisocyanat, oder Isophorondiisocyanat zum Einsatz. Bei der Herstellung im technischen Maßstab kommt es naturgemäß zu geplanten Abschaltungen oder auch ungeplanten Ausfällen des Prozesses. Die Gründe hierfür sind vielfältig und reichen vom Ausfall von Apparaten oder Steuerungseinrichungen über Störungen in der Rohstoffversorgung bis hin zu wirtschaftlichen Gründen wie einer zu schwachen Nachfrage nach dem Produkt.

Gerade ungeplante Ausfälle bereiten Probleme, weil in hierbei oft weitere Sicherheitsmaßnahmen greifen, um die Anlage in einen sogenannten sicheren Zustand zu bringen. Dazu gehört beispielsweise das Abschalten von Energiequellen. Da viele technisch relevante Diamine Schmelztemperaturen zwischen 5 °C und 105 °C aufweisen, neigen sie in solchen Fällen zur Erstarrung. Dies trifft nicht nur, aber vor allem auf den Einsatz der Amine in Freianlagen zu, in denen im Winter tiefe Umgebungstemperaturen herrschen. Einmal erstarrt, verursacht das Aufschmelzen des Diamins in Apparaten und Rohrleitungen einen erheblichen Mehraufwand bei der Wiederinbetriebnahme einer Anlage nach einem Stillstand oder bei der Reinigung entsprechender Apparate.

Die WO2015/144658 A1 befasst sich ausdrücklich mit Abweichungen vom Normalbetrieb bei der Phosgenierung von Aminen und gibt Hinweise darauf, wie bei solchen Abweichungen das Risiko von Ablagerungen in der Mischzone zur Vermischung von Amin und Phosgen beziehungsweise in der strömungstechnisch danach angeordneten Reaktionszone minimiert werden kann. Zu diesem Zweck wird empfohlen, stets einen Überschuss an Phosgen gegenüber dem Amin sicherzustellen, indem beispielsweise beim Anfahren der kontinuierlichen Produktion der Reaktor zunächst nur mit Lösungsmittel und Phosgen aufgeheizt und erst dann die Aminzufuhr gestartet wird, beziehungsweise beim Abfahren der kontinuierlichen Produktion zunächst die Aminzufuhr und erst dann die Phosgenzufuhr unterbrochen wird. Auch in den der Mischzone und in der Reaktionszone vorgelagerten Apparaten, die der Vorbereitung des Amins, also beispielsweise dessen Temperierung, Transport, Verdampfung oder Überhitzung, dienen, gibt es jedoch bei einem Anlagenstillstand zuvor genannte Probleme, auf die in diesem Dokument nicht eingegangen wird.

Die WO2010/100221 A1 beschreibt ein Verfahren zur Herstellung von Isocyanaten durch Umsetzung der korrespondierenden Amine in der Gasphase. Dabei werden Probleme durch Feststoffablagerungen zwischen dem Verdampfer und dem Mischorgan, also auf dem Weg, den das Amin gasförmig passiert, vermieden, indem die Temperatur von mit dem gasförmigen Amin in Kontakt stehenden Oberflächen oberhalb der Taupunktsgrenze des Amin enthaltenden Gasstroms gehalten wird. Auf Probleme mit Feststoffablagerungen in weiter stromaufwärts gelegenen Rohrleitungen und Apparaten, die das Amin in flüssiger Form passiert, wird nicht eingegangen und die beschriebenen Maßnahmen sind auch nicht geeignet, hier Abhilfe zu schaffen.

Es besteht daher ein Bedarf nach einem Verfahren und einer Vorrichtung zur Herstellung von Isocyanaten, die die genannten Probleme möglichst weitgehend vermeiden.

Diesem Bedarf Rechnung tragend ist ein Gegenstand der vorliegenden Erfindung ein Verfahren zur Herstellung von Isocyanaten durch Phosgenierung von Aminen mit Phosgen, bei dem das Amin in einer Aminvorlage, die über wenigstens eine Rohrleitung mit einem Reaktor verbunden ist, in flüssiger Form bereitgestellt wird, wobei der Rohrleitung gegebenenfalls als weitere Apparate ein oder mehrere Fördereinrichtungen, Sensoren, Aktoren, Wärmetauscher und/oder Mischeinrichtungen zugeordnet sind, und das Amin aus der Aminvorlage durch die Rohrleitung und gegebenenfalls dieser Rohrleitung zugeordnete Apparate in den Reaktor transferiert und im Reaktor mit einem Überschuss an Phosgen zum Isocyanat umgesetzt wird,
**dadurch gekennzeichnet,** dass die Temperatur von mit dem flüssigen Amin in Kontakt stehenden Oberflächen der Aminvorlage, der Rohrleitung, der Fördereinrichtung und der gegebenenfalls vorhandenen Apparate, im Normalbetrieb wenigstens 5 K oberhalb der Schmelztemperatur des Amins gehalten wird, wobei mindestens eines der folgenden Merkmale erfüllt ist:
a) Beheizen der Aminvorlage
b) Beheizen des Amins
c) Beheizen der Rohrleitung und/oder gegebenenfalls dieser zugeordneter Apparate
d) Isolieren der Aminvorlage
e) Isolieren der Rohrleitung und/oder gegebenenfalls dieser zugeordneter Apparate.

Das Wort "*ein"* ist im Rahmen dieser Erfindung im Zusammenhang mit zählbaren Größen lediglich als unbestimmter Artikel und nur dann als Zahlwort zu verstehen, wenn dies ausdrücklich, etwa durch den Zusatz "*genau* ein" gesagt wird. Ausdrücke wie "ein Wärmetauscher" oder "ein Aminstrom" schließen also die Möglichkeit des Vorhandenseins weiterer Wärmetauscher oder Aminströme nicht aus.

Bevorzugt bedeuten erfindungsgemäß die Ausdrücke "umfassend" oder "enthaltend", "im Wesentlichen bestehend aus" und besonders bevorzugt "bestehend aus".

Unter "Normalbetrieb" ist vorliegend zu verstehen, dass sich die Vorrichtung im Produktionsmodus befindet, also im Reaktor eine Reaktion von Amin mit Phosgen erfolgt und die Vorrichtung nicht aufgrund einer Störung in einen sicheren Zustand versetzt wurde oder aus anderen Gründen, beispielsweise zu Wartungszwecken oder wegen eines Kampagnen- oder Produktwechsels abgefahren wurde.

Sofern die Temperatur von mit dem flüssigen Amin in Kontakt stehenden Oberflächen nicht direkt gemessen wird oder werden kann, ist vorliegend anzunehmen, dass die Temperatur der Oberfläche der Temperatur des mit der Oberfläche in Kontakt stehenden Amins entspricht.

Dies ist gerade für durchströmte Rohrleitungen oder Behälter, in denen das Amin umgewälzt wird, eine zweckmäßige und hinreichend genaue Näherung. Allgemein ist es zur Durchführung des erfindungsgemäßen Verfahrens sinnvoll, Toträume in denen das Amin nicht bewegt wird, zu vermeiden. Dies kann beispielsweise erreicht werden, indem Vorlage- oder Zwischenbehälter mit einem Rührwerk oder einer Umpumpvorrichtung ausgestattet werden und zum Beispiel auf Stichleitungen möglichst verzichtet wird.

Unter der "Schmelztemperatur des Amins" ist vorliegend die Temperatur zu verstehen, oberhalb der das Amin als homogene flüssige Phase vorliegt und bei deren Unterschreitung diese zu erstarren beginnt. Für Reinstoffe oder eutektische Gemische ist diese Temperatur identisch mit der Temperatur am Schmelzpunkt. Sofern das Amin ein Schmelzintervall (auch als Schmelzbereich oder Erstarrungsintervall bezeichnet) aufweist, weil es beispielsweise als Isomerengemisch vorliegt, ist vorzugsweise die Liquidustemperatur des Gemisches, also die obere Grenze des Schmelzintervalls, als "Schmelztemperatur des Amins" heranzuziehen. Dabei handelt es sich wiederum die Temperatur, oberhalb der das Amin bzw. Amingemisch als homogene flüssige Phase vorliegt und bei deren Unterschreitung das Amin bzw. Amingemisch aus einer homogenen flüssigen Phase zu erstarren beginnt.

Für die meisten Amine liegen in der Literatur zuverlässige Angaben über deren Schmelztemperatur vor. Im Zweifelsfall kann die "Schmelztemperatur des Amins" im Rahmen der vorliegenden Erfindung vorzugsweise mittels der einer Kapillarmethode mit hinreichender Genauigkeit bestimmt werden. Entsprechende Vorgehensweisen sind dem Fachmann geläufig. Die bei Bedarf gefrorene und pulverisierte Substanz wird 2 bis 5 mm hoch in ein Schmelzpunktröhrchen gefüllt und verdichtet, z.B. indem das Schmelzpunktröhrchen durch ein Glasrohr auf eine feste Unterlage fallen gelassen wird. Bei hygroskopischen oder sublimierenden Stoffen wird das Röhrchen am oberen Ende zugeschmolzen. Im Bereich der zu erwartenden Schmelztemperatur, die sich aus Literaturangaben oder einer zuvor durchgeführten Grobbestimmung ergibt, wird mit einer niedrigen Heizrate von maximal 1 K pro Minute vorgegangen. Abgelesen wird die Temperatur, bei der das letzte feste Teilchen in die flüssige Phase übergeht.

"Isolieren" oder "Isolierung" beziehen sich vorliegend auf die Wärmedämmung von Apparaten oder Rohrleitungen, also auf das Reduzieren des Wärmedurchgangs durch Aufbringen von isolierenden Materialien wie beispielsweise Kautschuk, Schaumstoff oder Mineralwolle. Mineralwolle wird aufgrund der hohen Temperaturbeständigkeit und des geringen Brandrisikos in der Regel bevorzugt. Meist umfasst die Isolierung auf der Außenseite noch eine metallische Ummantelung, beispielsweise aus verzinktem Blech oder Aluminiumfolie. Durch das Aufbringen einer Isolierung werden also die Wärmeverluste minimiert, so dass die Abkühlung des Amins durch teilweise unvermeidbare Wärmebrücken oder fehlende Beheizung langsamer erfolgt, so dass der Temperaturabstand zum Schmelzpunkt kleiner gewählt werden kann oder bei gleichem Abstand mehr Zeit zur Verfügung steht, bevor Probleme auftreten.

Die vorliegende Erfindung betrifft ein Verfahren zur Herstellung von Isocyanaten, wobei das Amin bevorzugt eine Schmelztemperatur oberhalb von 5 °C aufweist. Bevorzugt ist das Amin ein Diamin, besonders bevorzugt ausgewählt aus der Gruppe bestehend aus 1,6-Diaminohexan (HDA), 1-Amino-3,5,5-trimethyl-5-aminomethylcyclohexan (IPDA), 1,5-Diaminopentan (PDA), 1,3-Bis(aminomethyl)benzol (m-XDA), 2,4-Diaminotoluol (2,4-TDA), 2,6-Diaminotoluol (2,6-TDA), 4,4'-Diaminodiphenylmethan (4,4'-MDA), 2,4'-Diaminodiphenylmethan (2,4'-MDA), 2,2'-Diaminodiphenylmethan (2,2'-MDA), 4,4'-Diaminodicyclohexylmethan (PACM), 1,4-Diaminobenzol (pPDA), 3,3'-Dimethyl-4,4'-4,4'-biphenyldiamin und 1,5-Diaminonaphthalin (NDA). Es können auch Mischungen solcher Amine zur Herstellung der entsprechenden Isocyanate eingesetzt werden. Ganz besonders bevorzugt ist das Amin ausgewählt aus der Gruppe bestehend aus 1,6-Diaminohexan (HDA), 2,4-Diaminotoluol (2,4-TDA), 2,6-Diaminotoluol (2,6-TDA), 4,4'-Diaminodiphenylmethan (4,4'-MDA), 2,4'-Diaminodiphenylmethan (2,4'-MDA), 2,2'-Diaminodiphenylmethan (2,2'-MDA), 4,4'-Diaminodicyclohexylmethan (PACM), 1,4-Diaminobenzol (pPDA), 3,3'-Dimethyl-4,4'-4,4'-biphenyldiamin und 1,5-Diaminonaphthalin (NDA).

Die Herstellung der Isocyanate kann durch Phosgenierung von Aminen nach verschiedenen, dem Fachmann bekannten Verfahren des Standes der Technik durchgeführt werden. Die Phosgenierung kann kontinuierlich, semikontinuierlich oder diskontinuierlich erfolgen. Bevorzugt erfolgt die Phosgenierung als kontinuierliches Verfahren.

Die Phosgenierung kann sowohl in der Gasphase als auch in flüssiger Phase durchgeführt werden. Bei der Phosgenierung in flüssiger Phase kann das Amin als solches mit Phosgen zur Reaktion gebracht werden (Basenphosgenierung) oder es wird zunächst, beispielsweise durch Umsetzung mit Chlorwasserstoff oder Kohlendioxid in ein Salz überführt und erst in dieser Form mit Phosgen umgesetzt.

Erfolgt die Phosgenierung in der Gasphase, so wird das Amin mit Hilfe der Fördereinrichtung aus der Aminvorlage entnommen und in der Regel vor dem Eintritt in den Reaktor in einem geeigneten Verdampfer in die Gasphase überführt. Bevorzugt erfolgt nach dieser Verdampfung noch eine Überhitzung des gasförmigen Aminstroms auf eine Temperatur, die wenigstens 5 K oberhalb der Kondensationstemperatur des Amins unter den vorherrschenden Bedingungen, insbesondere des vorherrschenden Drucks, liegt. Gewöhnlich liegt diese Temperatur bei 200 °C bis 600 °C, bevorzugt bei 250 °C bis 500 °C und besonders bevorzugt bei 250 °C bis 330 °C.

Erfolgt die Phosgenierung in flüssiger Phase, so wird diese in der Regel in Gegenwart eines Lösungsmittels, beispielsweise Chlorbenzol, Dichlorbenzol oder einer Mischung dieser beiden Lösungsmittel, durchgeführt. Wie zuvor ausgeführt, kann das Amin direkt mit Phosgen umgesetzt werden oder zunächst beispielsweise mit Chlorwasserstoffgas oder Kohlendioxid unter Bildung der entsprechenden Salze umgesetzt und dann phosgeniert werden. Auch diese Salzbildung erfolgt dann in der Regel bereits in einem inerten Lösungsmittel, wobei eine Suspension des Salzes erhalten wird.

Unabhängig vom angewendeten Phosgenierverfahren wird das Amin im erfindungsgemäßen Verfahren zunächst in einer Aminvorlage in flüssiger Form bereitgestellt. Der Begriff "flüssige Form" ist dabei so zu verstehen, dass das entsprechende Amin flüssig vorliegt. Geringe Anteile von Schwebstoffen, beispielsweise durch Verunreinigungen lassen sich nicht immer vollständig vermeiden und sind hierbei mit umfasst. Bei der Aminvorlage kann es sich um einen stationären oder einen mobilen Tank handeln, der zur Lagerung des jeweiligen Amins geeignet ist. Bevorzugt handelt es sich um einen stationären Tank. Die Aminvorlage ist über eine Rohrleitung mit dem Reaktor verbunden und durch diese kann das Amin aus der Aminvorlage in den Reaktor transferiert werden.

Der Rohrleitung können gegebenenfalls weitere Apparate zugeordnet sein. Dabei handelt es sich insbesondere um eine oder mehrere Fördereinrichtugnen, beispielsweise Pumpen, die dazu dienen, das Amin durch die Rohrleitung zum Reaktor zu fördern. Für eine exakte Dosierung des Amins und eine Überwachung und/oder Kontrolle der Prozessbedingungen ist es förderlich, wenn die Rohrleitung über Sensoren wie beispielsweise Massendurchflussmesser, Druckaufnehmer und/oder Temperaturfühler sowie über Aktoren wie beispielsweise Regelventile, verfügt. Darüber hinaus können der Rohrleitung Mischeinrichtungen wie beispielsweise Statikmischer oder dynamische Mischaggregate zugeordent sein. Diese können dazu genutzt werden, um das Amin beispielsweise mit anderen Aminen, Lösungsmitteln oder Hilfsstoffen zu vermischen. Dynamische Mischaggregate sind dabei üblicherweise in Mischbehältern angeordnet, die dann gegebenenfalls ebenfalls der Rohrleitung zuzuordnen sind.

Vor allem, aber nicht nur für den Fall der Gasphasenphosgenierung können der Rohrleitung auch Wärmetauscher oder Elektroerhitzer zugeordnet sein, mit deren Hilfe das Amin auf eine gewünschte Temperatur erwärmt wird. Die Wärmetauscher können beispielsweise mit Dampf oder Wärmeträgeröl betrieben werden. Eine attraktive Möglichkeit ist auch der Betrieb mit einem zu kühlenden, warmen Prozessstrom, dem Wärme entzogen und an das Amin abgegeben wird. Durch eine derartige Energieintegration kann Heiz- und Kühlenergie eingespart werden. Sofern z.B. für eine anschließende Gasphasenphosgenierung das Amin in einem Wärmetauscher verdampft wird, wird dieser üblicherweise als Verdampfer bezeichnet. Wird das erhaltene, gasförmige Amin weiter erhitzt, spricht man von Überhitzung und entsprechende Wärmetauscher werden auch als Überhitzer bezeichnet. Bei entsprechender Auslegung des Verdampfers kann die Überhitzung auch direkt im Verdampfer erfolgen.

Gerade bei kontinuierlichen Verfahren besteht eine hohe Wahrscheinlichkeit, dass es beim längeren Betrieb zu ungeplanten Produktionsunterbrechungen kommt. Zu den üblichen Maßnahmen in einem solchen Fall gehört es nicht nur, die Dosierung der Einsatzstoffe in den Prozess zu beenden, sondern in der Regel auch, Energiequellen abzuschalten. Dies hat zur Folge, dass sich das in der Aminvorlage und in der Rohrleitung zum Reaktor befindliche Amin abkühlt. Wird dabei der Festpunkt des Amins unterschritten, kommt es regelmäßig zu Ablagerungen von festem Amin, die beim späteren Wiederanfahren der Phosgenierungsanlage zu Problemen führen. Durch Ablagerungen verengte Rohrleitungsabschnitte können verhindern, dass der erforderliche Durchsatz erreicht wird oder es kann gar zur kompletten Verstopfung von Rohrleitungsabschnitten kommen. Auch in der Aminvorlage kann es zu Ablagerungen kommen, so dass gegebenenfalls Wärmeübergänge verschlechtert werden oder Rühraggregate feststecken können. Das Aufschmelzen derartiger Amin-Ablagerungen und damit die sichere Wiederinbetriebnahme bedeutet einen erheblichen Mehraufwand für den Anlagenbetreiber. Dies gilt umso mehr, wenn es wegen der Verstopfungen bereits nicht mehr möglich ist, Rohrleitungen mit heißem Amin zu durchströmen und so Ablagerungen aufzuschmelzen. Unter Umständen besteht dann aufgrund der thermischen Expansion beim Aufschmelzen sogar ein Sicherheitsrisiko.

Um diese Probleme zu verhindern, wird erfindungsgemäß dafür Sorge getragen, dass die Temperatur von mit dem flüssigen Amin in Kontakt stehenden Oberflächen der Aminvorlage, der Rohrleitung und der gegebenenfalls vorhandenen, der Rohrleitung zugeordneten Apparate, wenigstens 5 K, bevorzugt wenigstens 10 K, besonders bevorzugt wenigstens 20 K und ganz besonders bevorzugt wenigstens 30 K oberhalb der Schmelztemperatur des Amins gehalten wird. Dabei ist es nicht erforderlich, dass alle genannten Oberflächen die gleiche Temperatur aufweisen. Bevorzugt sind somit entlang der Strömungsrichtung in Stufen steigende Oberflächen- und damit letztlich auch Amintemperaturen. Auf diese Weise bleibt das Amin in der Aminvorlage, in der üblicherweise eine hohe Verweilzeit vorliegt, vergleichsweise kühl und wird erst kurz vor der Reaktion auf die eigentliche Starttemperatur erwärmt, so dass die thermische Belastung des Rohstoffs trotz der erfindungsgemäß über die Schmelztemperatur erhöhten Temperaturen gering bleibt und letztlich auch Energieverluste durch große heiße Oberflächen nach außen geringgehalten werden.

Durch den erfindungsgemäßen Abstand der Temperatur der mit dem flüssigen Amin in Kontakt stehenden Oberflächen von der Schmelztemperatur des Amins wird gewährleistet, dass im Falle eines ungeplanten Stillstands genügend Zeit verbleibt, geeignete Gegenmaßnahmen zu ergreifen, bevor die Temperatur des Amins in der Aminvorlage und/oder in der Rohrleitung bzw. in dieser zugeordneten Apparaten unter die Schmelztemperatur fällt und Probleme durch erstarrtes Amin in der Aminvorlage und/oder der Rohrleitung bzw. in den dieser Rohrleitung zugeordneten Apparate auftreten. Geeignete Gegenmaßnahmen können beispielsweise die Behebung der ursächlichen Störung für den ungeplanten Stillstand sein, so dass der Prozess wieder angefahren werden kann, bevor weitere Probleme auftreten. Eine weitere mögliche Gegenmaßnahme ist es, im Falle eines geplanten oder ungeplanten Abfahrens der der Phosgenierung, das Amin zumindest teilweise aus einem oder mehreren aminführenden Anlagenteilen zu entfernen, wobei das entfernte Amin vorzugsweise in einen Sammelbehälter transferiert wird. Vorzugsweise handelt es sich bei dem Sammelbehälter um die Aminvorlage. Eine weitere mögliche Gegenmaßnahme ist die Implementierung einer behelfsmäßigen externen Beheizung kritischer aminführender Anlagenteile oder auch einer Zirkulation des Amins, wobei vorzugsweise ein Eintrag von Wärmeenergie in das Amin erfolgt, der ein weiteres Absinken der Temperatur unter die Schmelztemperatur des Amins verhindert.

Auch für den Fall, dass lediglich die Temperierung des Amins ausfällt, bietet das erfindungsgemäße Verfahren den Vorteil, dass der Prozess noch für eine gewisse Zeit weiterbetrieben werden kann, die dann genutzt werden kann, die Störung der Temperierung zu beheben.

Da es sich bei einigen organischen Aminen um thermisch empfindliche Substanzen handelt, die nicht über einen längeren Zeitraum bei zu hoher Temperatur gehalten werden sollten, ist es insbesondere für die Aminvorlage empfehlenswert, wenn die Temperatur der mit dem flüssigen Amin in Kontakt stehenden Oberflächen 180 °C, bevorzugt 150 °C und besonders bevorzugt 120 °C nicht überschreitet. Es versteht sich von selbst, dass die Einhaltung einer solchen maximalen Oberflächentemperatur im erfindungsgemäßen Verfahren nur für solche Amine möglich ist, deren Schmelzpunkt hinreichend weit unterhalb dieser maximalen Oberflächentemperatur liegt. Für höher schmelzende Amine ist es bevorzugt, auch höhere Oberflächentemperaturen, beispielsweise bis zu 200 °C oder 220 °C zuzulassen, die wenigstens 5 K oberhalb der Schmelztemperatur des Amins liegen.

Um das erfindungsgemäße Ziel zu erreichen, dass die Temperatur von mit dem flüssigen Amin in Kontakt stehenden Oberflächen der Aminvorlage, der Rohrleitung, der Fördereinrichtung und der gegebenenfalls vorhandenen Apparate, im Normalbetrieb wenigstens 5 K oberhalb der Schmelztemperatur des Amins gehalten wird, wird wenigstens eine der folgenden Maßnahmen umgesetzt:
a) Beheizen der Aminvorlage
b) Beheizen des Amins
c) Beheizen der Rohrleitung und/oder gegebenenfalls dieser zugeordneter Apparate
d) Isolieren der Aminvorlage
e) Isolieren der Rohrleitung und/oder gegebenenfalls dieser zugeordneter Apparate.

In einer bevorzugten Ausführungsform der Erfindung werden zwei oder mehr dieser Maßnahmen in beliebiger Kombination umgesetzt.

In einer weiteren bevorzugten Ausführungsform der Erfindung wird wenigstens eine der Maßnahmen a) Beheizen der Aminvorlage, b) Beheizen des Amins oder c) Beheizen der Rohrleitung und/oder gegebenenfalls dieser zugeordneter Apparate in Kombination mit wenigstens einer der Maßnahmen d) Isolieren der Aminvorlage oder e) Isolieren der Rohrleitung und/oder gegebenenfalls dieser zugeordneter Apparate umgesetzt.

In einer weiteren bevorzugten Ausführungsform der Erfindung werden wenigstens die Maßnahmen a) und d) und/oder die Maßnahmen c) und e) in Kombination miteinander umgesetzt, so dass jeweils Apparate oder Rohrleitungsabschnitte, die beheizt werden, auch isoliert werden.

In einer besonders bevorzugten Ausführungsform der Erfindung werden wenigstens die Maßnahmen a), c), d) und e) umgesetzt.

Ein Beheizen der Aminvorlage kann auf verschiedenen Wegen erfolgen. Es kann beispielsweise eine Doppelmantelausführung der Aminvorlage gewählt werden, so dass die Beheizung durch Zirkulation eines Wärmeträgermediums oder mittels Dampf im Zwischenraum des Doppelmantels erfolgen kann. Weiterhin ist es auch möglich, an der Außen- oder Innenseite der Aminvorlage eine Heizschlange, vorzugsweise eine Halbrohrheizschlange aufzubringen und diese mit einem entsprechend temperierten Wärmeträgermedium oder einem anderen warmen Prozessmedium zu durchströmen. Eine weitere Möglichkeit ist die Beheizung über einen außen angebrachten elektrischen Heizmantel. Je nach lokalen Gegebenheiten gibt es weitere Möglichkeiten, die Aminvorlage zu beheizen, beispielsweise die Beaufschlagung mit heißer Luft oder IR-Strahlung, auf die hier jedoch nicht im Einzelnen eingegangen wird. Wichtig ist lediglich, dass die Beheizung derart eingerichtet ist, dass sich damit die erfindungsgemäße Oberflächentemperatur der mit dem flüssigen Amin in Kontakt stehenden Oberflächen erzielen lässt.

Ein Beheizen des Amins kann auf verschiedenen Wegen erfolgen. Es kann beispielsweise mittels eines in die Aminvorlage integrierten Heizregisters erfolgen, das beispielsweise durch ein Wärmeträgermedium, kondensierenden Dampf oder elektrisch beheizt wird. Möglich ist auch eine Beheizung mittels Mikrowellenstrahlung. Eine vorteilhafte Methode ist es, einen Teil des Amins aus der Vorlage zu entnehmen, mit Hilfe eines Wärmetauschers zu erhitzen und dann in die Aminvorlage zurückzuführen. Durch einen solchen Umpumpvorgang findet gleichzeitig eine Durchmischung des Amins statt. Die Erwärmung des Amins kann in dieser Ausführung sehr kontrolliert erfolgen und lässt sich besonders einfach energetisch z.B. durch Wärmeintegration mit anderen Prozessströmen optimieren.

Ein Beheizen der Rohrleitung kann auf verschiedenen Wegen erfolgen. Üblich ist die Verwendung von Begleitheizungen. Diese können beispielsweise mittels elektrischer Heizbänder ausgeführt werden, vorzugsweise mittels selbstregulierender elektrischer Heizbänder, die den Leistungseintrag automatisch je nach Oberflächentemperatur anpassen. Alternativ können auch Fluidbegleitheizungen zum Einsatz kommen. Dabei werden Schläuche oder kleine Rohrleitungen verwendet, die z.B. mittels Heißwasser, Dampf oder einem Wärmeträger wie Glykol beheizt werden. Alternativ zu einer solchen Begleitheizung können Rohrleitungen oder Rohrleitungsabschnitte auch komplett doppelwandig ausgeführt und über den Mantelraum beheizt werden. Begleitheizungen bieten jedoch viele Vorteile. So sind die Installation und Wartung relativ einfach und Leckagen an der eigentlichen Rohrleitung können einfach erkannt und repariert werden.

Es ist nicht zwingend erforderlich, aber bevorzugt, die Rohrleitung auf ihrer kompletten Strecke zu beheizen oder komplett mit derselben Methode zu beheizen. Es kann je nach lokalen Gegebenheiten vielmehr sinnvoll sein, Abschnitte der Rohrleitung nicht zu beheizen oder mittels unterschiedlicher Methoden zu beheizen. Sofern nicht beheizte Rohrleitungsabschnitte vorliegen, ist es zweckmäßig, diese mit einer Isolierung zu versehen, um lokale Kaltstellen zu vermeiden.

Durch die Isolierung der Aminvorlage, der Rohrleitung und/oder gegebenenfalls weiterer, der Rohrleitung zugeordneter Apparate lassen sich die Wärmeverluste minimieren. Dies führt im Störungsfall zu einer langsameren Abkühlung des darin befindlichen Amins und ermöglicht es so, den erfindungsgemäß notwendigen Abstand der Oberflächentemperatur von der Schmelztemperatur des Amins gering zu halten. Auf diese Weise lassen sich Zersetzung des Amins an heißen Oberflächen sowie der Energiebedarf für das Verfahren minimieren. Ein großer Vorteil der Isolierung ist, dass sie nicht wie beispielsweise eine Begleitheizung von einer externen Energiezufuhr abhängig ist. Ihre Funktion und damit die zuvor genannte Vorteile bleiben deshalb auch im Störungsfall vorhanden.

Als Materialien für die Isolierung der Aminvorlage, der Rohrleitung und/oder der gegebenenfalls dieser Rohrleitung zugeordneten Apparate eignen sich beispielsweise Kautschuk, Schaumstoff oder Mineralwolle. Mineralwolle wird aufgrund der hohen Temperaturbeständigkeit und des geringen Brandrisikos in der Regel bevorzugt. Meist umfasst die Isolierung auf der Außenseite noch eine metallische Ummantelung, beispielsweise aus verzinktem Blech oder Aluminiumfolie.

Durch das erfindungsgemäße Verfahren ist gewährleistet, dass im Fall einer Produktionsunterbrechung genügend Zeit bleibt, um die Ursache für die Unterbrechung zu beheben oder andere Gegenmaßnahmen zu ergreifen, die geeignet sind, spätere Probleme durch verfestigtes Amin in den Anlagenteilen oder Rohrleitungen zu minimieren oder nach Möglichkeit zu vermeiden. Bevorzugt dauert es im Falle einer Produktionsunterbrechung aufgrund der erfindungsgemäßen Vorgehensweise wenigstens 2 Minuten, besonders bevorzugt wenigstens 5 Minuten, ganz besonders bevorzugt wenigstens 15 Minuten und am meisten bevorzugt wenigstens 30 Minuten, bevor das Amin aufgrund von Wärmeverlusten seine Schmelztemperatur unterschreitet und sich verfestigt.

Ein weiterer Gegenstand der Erfindung ist die Verwendung mindestens einer Heizvorrichtung und mindestens einer Isolierung mit wenigstens einem Temperatursteuerungssystem oder Temperaturregelungssystem, zur Bewahrung des flüssigen Zustands eines Amins in einem Verfahren zur Herstellung von Isocyanaten durch Phosgenierung von Aminen über wenigstens 2 Minuten, bevor das Amin aufgrund von Wärmeverlusten seine Schmelztemperatur unterschreitet und sich verfestigt.

Hierbei ist es weiter bevorzugt, dass der flüssige Zustand des Amins wenigstens 5 Minuten, bevorzugt wenigstens 15 Minuten und besonders bevorzugt wenigstens 30 Minuten bewahrt wird. Alternativ oder zusätzlich bevorzugt ist die erfindungsgemäße Verwendung dadurch gekennzeichnet, dass die Bewahrung des flüssigen Zustands während einer Produktionsunterbrechung stattfindet.

Bei Verfahren zur Herstellung von Isocyanaten in der flüssigen Phase wird häufig in einem ersten Schritt eine Lösung des Amins in einem inerten Lösungsmitte wie beispielsweise Chlorbenzol, Dichlorbenzol oder einer Mischung aus beiden hergestellt. Dies erfolgt üblicherweise in einem Mischbehälter, der im Sinne der vorliegenden Erfindung als der Rohrleitung zugeordneter Apparat anzusehen ist. Der Weg des Amins vom Mischbehälter zum Reaktor ist dann bezüglich der Temperaturen weniger kritisch, da durch geeignete Wahl des Lösungsmittels ein Ausfrieren des Amins und damit eine Kristallisation verhindert werden kann. Es ist also bevorzugt insbesondere in der Aminvorlage und/oder in dem Rohrleitungsabschnitt bis zum Mischbehälter, in dem die Aminlösung hergestellt wird, auf das Einhalten des erfindungsgemäßen Temperaturabstands zwischen den mit dem flüssigen Amin in Kontakt stehenden Oberflächen und der Schmelztemperatur des Amins zu achten.

Die Erfindung betrifft ferner eine Phosgenierungsanlage zur Durchführung des erfindungsgemäßen Verfahrens, umfassend oder bestehend aus
I) mindestens eine Aminvorlage zur Bereitstellung eines flüssigen Aminstroms,
II) mindestens eine Phosgenstrom-Vorrichtung zur Bereitstellung eines Phosgenstroms,
III) einen Reaktor zur Vermischung der Ströme aus (I) und (II) und Umsetzung des Phosgenstroms mit dem Aminstrom,
IV) wobei von der Aminvorlage mindestens eine Rohrleitung zur Beförderung des zumindest zunächst flüssigen Amins zum Reaktor abgeht,
   dadurch gekennzeichnet, dass die Phosgenierungsanlage wenigstens ein Temperatursteuerungssystem oder Temperaturregelungssystem umfasst, das eines oder mehrere Heizvorrichtungen zur direkten und/oder indirekten Erwärmung des Amins aufweist.

Die eine oder mehrere Heizvorrichtung der erfindungsgemäßen Phosgenierungsanlage ist vorzugsweise dazu eingerichtet eine Temperaturdifferenz von wenigstens 5 K, bevorzugt wenigstens 10 K, besonders bevorzugt wenigstens 20 K und ganz besonders bevorzugt wenigstens 30 K oberhalb der Schmelztemperatur des Amins zu halten. Bei der Aminvorlage handelt es sich beispielsweise um einen stationären oder einen mobilen Tank, der zur Lagerung des jeweiligen Amins geeignet ist. Bevorzugt handelt es sich um einen stationären Tank. Vorzugsweise umfasst die Aminvorlage eine Einrichtung zur Umwälzung des flüssigen Amins, beispielsweise ein Rührwerk oder eine Umpumpvorrichtung.

Die Phosgenstrom-Vorrichtung kann weitere Apparate umfassen, die für den Betrieb der Phosgenierungsanlage dienlich sind, wie beispielsweise Wärmetauscher, Einrichtungen zur Rückgewinnung und Aufbereitung von überschüssigem Phosgen, Misch- und Dosiereinrichtungen zur Herstellung und Dosierung eines gemischten Phosgens aus rezykliertem und frischem Phosgen, sowie Messeinrichtungen zur Bestimmung von Prozessparametern wie beispielsweise Druck oder Temperatur des Phosgenstroms. Die Phosgenstrom-Vorrichtung ist über mindestens eine Zuleitung mit dem Reaktor verbunden. Die Verbindung kann beispielsweise über einfache Rohrleitungen und Flanschverbindungen erfolgen.

Reaktoren, die zur Umsetzung des Phosgenstroms mit dem Aminstrom eingesetzt werden können, sind dem Fachmann bekannt. Es eignen sich Verweilzeitreaktoren mit einer vorgeschalteten oder integrierten Mischeinrichtung. Geeignete Verweilzeitreaktoren sind beispielsweise Rohrreaktoren oder Rührkesselkaskaden, bevorzugt Rohrreaktoren. Vorzugsweise erfolgt in Rohrreaktoren die Vermischung der beiden Ströme mit Hilfe von Glattstrahldüsen oder Ringspaltdüsen. Besonders bevorzugt ist dabei die Düse innerhalb des Reaktors angeordnet und mit der Rohrleitung verbunden, durch die das Amin aus der Aminvorlage bereitgestellt wird.

Ein Temperatursteuerungssystem ist vorliegend ein System, das dazu eingerichtet ist, die Temperatur des Amins direkt oder indirekt zu beeinflussen. Das Temperatursteuerungssystem hat dabei einen offenen Wirkungsablauf, d.h. es erfolgt keine Rückmeldung an die Steuerungseinheit, es können also durch Störgrößen Temperaturänderungen hervorgerufen werden. Dies kann bei Durchführung des erfindungsgemäßen Verfahrens beispielsweise berücksichtigt werden, indem ein größerer Temperaturabstand anvisiert wird oder indem mögliche Störgrößen selbst überwacht werden und in der Ermittlung eines Stellwerts für die Heizvorrichtung berücksichtigt werden. Bevorzugt ist jedoch die Verwendung eines Temperaturregelungssystems, bei dem die Regelgröße gemessen wird und in einen geschlossenen Wirkkreis einfließt.

In einer bevorzugten Ausführungsform weist demnach die Phosgenierungsanlage ein Temperaturregelungssystem auf, das mindestens einen Temperatursensor zur Messung der Temperatur des Amins in der Aminvorlage und mindestens eine Steuereinheit, die mit dem Temperatursensor und einem oder mehreren Heizvorrichtungen in Verbindung steht, umfasst, wobei die Steuereinheit zur Regelung der Temperatur des flüssigen Amins in der Aminvorlage, bevorzugt durch Steuerung der Leistung der Heizvorrichtungen, eingerichtet ist.

Geeignete Heizvorrichtungen für die möglichen unterschiedlichen Anwendungsfälle sind dem Fachmann bekannt. Die Aminvorlage kann mit einem Doppelmantel ausgeführt sein, so dass die Beheizung durch ein geeignetes Heizmedium über den Zwischenraum des Doppelmantels erfolgen kann. Das Temperatursteuerungssystem oder Temperaturregelungssystem kann dann beispielsweise auf den Mengenstrom an Heizmedium oder die Temperatur des Heizmediums, in der Regel Dampf oder ein Wärmeträgeröl, wirken. Statt des Doppelmantels kann die Heizvorrichtung auch eine an der Außen- oder Innenseite der Aminvorlage aufgebrachte Heizschlange, vorzugsweise eine Halbrohrheizschlange aufweisen, die wiederum eingerichtet ist, Wärme aus einem entsprechend temperierten Wärmeträgermedium oder einem anderen warmen Prozessmedium an das Amin zu übertragen. Weitere Ausführungsformen der Heizvorrichtung umfassen beispielsweise Ölbäder, IR-Strahler, elektrische Heizbänder oder - mäntel, elektrische Heizstäbe, Gas- oder Ölbrenner. Eine Kombination aus Umwälzung und Beheizung lässt sich realisieren, wenn die Heizvorrichtung in eine Umpumpvorrichtung der Aminvorlage integriert wird. Hierzu kann beispielsweise die Aminvorlage über eine Ringleitung, umfassend eine Fördereinrichtung und wenigstens einen Wärmetauscher, verfügen. Der Wärmetauscher kann dann beispielsweise als Rohrbündelwärmetauscher, als doppelwandiges Rohr oder als Plattenwärmetauscher ausgeführt sein. Geeignete Heizvorrichtungen für die Rohrleitung sind beispielsweise elektrische Heizbänder, vorzugsweise selbstregulierende elektrische Heizbänder. Ebenfalls möglich sind Fluidbegleitheizungen, umfassend an der Rohrleitung positionierte Schläuche oder kleine Rohrleitungen, die eingerichtet sind, beispielsweise mittels Heißwasser, Dampf oder einem Wärmeträger wie Glykol beheizt zu werden.

Auch und gerade in Rohrleitungen kann es aufgrund von ungünstigen Verhältnissen zwischen Oberfläche und Volumen beim Unterschreiten des Schmelzpunkts des Amins rasch zu Verengungen bis hin zu Verstopfungen kommen. Kritische Bereiche sind dabei insbesondere auch Stellen mit Flanschverbindungen oder solche, an denen die Rohrleitung durch Schellen oder andere Halterungen befestigt ist, da diese häufig zusätzliche Wärmeverluste verursachen.

In einer weiteren bevorzugten Ausführungsform weist deshalb die Phosgenierungsanlage mindestens eine Heizvorrichtung auf, die der Rohrleitung zugeordnet ist, wobei die Heizvorrichtung entlang eines wesentlichen Teils der Rohrleitung verläuft und dazu eingerichtet ist, die Rohrleitung und damit indirekt das flüssige Amin im inneren der Rohrleitung zu erwärmen. Unter einem "wesentlichen Teil der Rohrleitung" sind vorliegend wenigstens 50%, bevorzugt wenigstens 80%, besonders bevorzugt wenigstens 90% und ganz besonders bevorzugt wenigstens 95% der Rohrstrecke zwischen der Aminvorlage und dem Reaktor zu verstehen.

Um auch bei einer Störung der Heizvorrichtung Probleme durch kristallisierendes Amin zeitlich hinauszuzögern, ist es bevorzugt, einen wesentlichen Teil der Rohrleitung mit einer Isolierung zu versehen. Auch hier ist unter einem "wesentlichen Teil der Rohrleitung" wenigstens 50%, bevorzugt wenigstens 80%, besonders bevorzugt wenigstens 90% und ganz besonders bevorzugt wenigstens 95% der Rohrstrecke zwischen der Aminvorlage und dem Reaktor zu verstehen. Insbesondere ist es empfehlenswert, Bereiche, in denen sich Halterungen, Flansche, Probenahmestellen oder andere potenziellen Wärmebrücken befinden mit einer Isolierung zu versehen. Als isolierenden Materialien, die auf die Rohrleitung aufgebracht werden können, eignen sich beispielsweise Kautschuk, Schaumstoff oder Mineralwolle. Mineralwolle wird aufgrund der hohen Temperaturbeständigkeit und des geringen Brandrisikos in der Regel bevorzugt. Es ist zweckmäßig, die Isolierung auf der Außenseite mit einer Schicht aus Kunststoff oder Metall zu ummanteln.

Soll die Umsetzung des Amins mit dem Phosgen zum Isocyanat in der Gasphase erfolgen, eignet sich hierfür vorzugsweise eine Phosgenierungsanlage gemäß einer der zuvor beschriebenen Ausführungsformen, dadurch gekennzeichnet, dass die Phosgenstrom-Vorrichtung einen gasförmigen Phosgenstrom bereitstellt, gegebenenfalls umfassend neben Phosgen einen Inertstoff im Phosgenstrom und die Vorrichtung mindestens eine Aminstrom-Vorrichtung zur Überführung des flüssigen Aminstroms in einen gasförmigen Aminstrom, gegebenenfalls umfassend neben Amin einen Inertstoff im Aminstrom, umfasst und gegebenenfalls mindestens eine Inertstoffstrom-Vorrichtung zur Bereitstellung eines Inertstoffstroms umfasst.

Die vorliegende Erfindung wird im Folgenden anhand von Ausführungsbeispielen erörtert, ist jedoch nicht auf diese Ausführungen beschränkt.

### Beispiele

Die Ausführung erfolgt in einer erfindungsgemäßen Phosgenierungsanlage zur Herstellung von Isocyanaten durch Phosgenierung entsprechender Amine. Diese Vorrichtung umfasst einen senkrecht aufgestellten Rohrreaktor (Phosgenierungsreaktor) zur Umsetzung des Amins mit Phosgen in der Gasphase. Außerdem umfasst die Vorrichtung eine Aminvorlage für das Amin, die über eine Rohrleitung fluidisch mit dem Rohrreaktor verbunden ist. Diese Vorlage ist doppelwandig ausgeführt, wobei durch den Zwischenraum ein temperiertes Wärmeträgeröl zirkuliert werden kann, um die Aminvorlage zu beheizen.

Für die erfindungsgemäßen Beispiele wird die Vorrichtung um eine außen aufgebrachte Isolierung der Aminvorlage sowie eine Begleitheizung und eine Isolierung der Rohrleitung ergänzt.

Das Wärmeträgeröl selbst kann mit Hilfe eines elektrischen Heizelements temperiert werden. Die Temperatur innerhalb der Aminvorlage wird mittels eines in die Vorlage eingebrachten Thermoelements erfasst und an eine Steuerungseinheit übermittelt, die aus den erfassten Daten einen Stellwert für das Heizelement errechnet und an dieses übermittelt, so dass die Temperatur in der Aminvorlage auf einen gewünschten Zielwert geregelt werden kann.

An einem Auslass der Aminvorlage ist eine Rohrleitung angeschlossen, die die Aminvorlage über eine Förderpumpe für das Amin, ein Regelventil zur Regelung des Massenstroms, einen Verdampfer und schließlich einen Überhitzer mit dem Rohrreaktor verbindet. Dort mündet die Rohrleitung in einer Düse aus der das verdampfte Amin austritt, mit Phosgen vermischt und in der Folge zum Isocyanat umgesetzt wird.

### Vergleichsbeispiel 1 (Herstellung von Toluol-2,4-diisocyanat)

In der Aminvorlage der zuvor beschriebenen Vorrichtung wird geschmolzenes 2,4-Diaminotoluol vorgelegt und die Temperatur in der Vorlage auf 100 °C geregelt. Zum Anfahren der Phosgenierung wird der Rohreaktor zunächst mit auf 330 °C vorgeheiztem Phosgen beaufschlagt, bis sich eine konstante Temperatur einstellt. Dann wird die Reaktion gestartet, indem mit der Dosierung von Amin begonnen wird. Das Amin wird mit Hilfe der Förderpumpe durch die mit der Aminvorlage verbundene Rohrleitung in den Verdampfer geleitet, dort verdampft und überhitzt und dann als überhitzter Dampf durch eine Düse in den Reaktor eingespeist. Der erforderliche Massenstrom an Amin wird mit Hilfe eines Regelventils in der Rohrleitung zwischen Förderpumpe und Verdampfer eingestellt. Nach einer Laufzeit von ca. 1 Stunde wird die Reaktion für ca. 15 Minuten unterbrochen, indem die Aminzufuhr und die Energiezufuhr des Heizelements gestoppt werden. Nach Ablauf der 15 Minuten gelingt es nicht, die Aminzufuhr wieder zu starten und auf den gewünschten Solldurchsatz einzuregeln.

### Vergleichsbeispiel 2 (PACM)

In der Aminvorlage der zuvor beschriebenen Vorrichtung wird geschmolzenes 4,4'-Diaminodicyclohexylmethan vorgelegt und die Temperatur in der Vorlage auf 66 °C geregelt. Zum Anfahren der Phosgenierung wird der Rohreaktor zunächst mit auf 360 °C vorgeheiztem Phosgen beaufschlagt, bis sich eine konstante Temperatur einstellt. Dann wird die Reaktion gestartet, indem mit der Dosierung von Amin begonnen wird. Das Amin wird mit Hilfe der Förderpumpe durch die mit der Aminvorlage verbundene Rohrleitung in den Verdampfer geleitet, dort verdampft und überhitzt und dann als überhitzter Dampf durch eine Düse in den Reaktor eingespeist. Der erforderliche Massenstrom an Amin wird mit Hilfe eines Regelventils in der Rohrleitung zwischen Förderpumpe und Verdampfer eingestellt. Nach einer Laufzeit von ca. 1 Stunde wird die Reaktion für ca. 15 Minuten unterbrochen, indem die Aminzufuhr und die Energiezufuhr des Heizelements gestoppt werden. Auch in diesem Beispiel gelingt es nach Ablauf der 15 Minuten nicht, die Aminzufuhr wieder zu starten und auf den gewünschten Solldurchsatz einzuregeln.

### Beispiel 1 (Herstellung von Toluol-2,4-diisocyanat)

Die zuvor beschriebene Vorrichtung wird um eine außen aufgebrachte Isolierung der Aminvorlage ergänzt. Außerdem wird an der Rohrleitung eine Begleitheizung und ebenfalls eine Isolierung angebracht. In der Aminvorlage der so modifizierten Vorrichtung wird geschmolzenes 2,4-Diaminotoluol vorgelegt und die Temperatur in der Vorlage auf 123 °C geregelt. Zum Anfahren der Phosgenierung wird der Rohreaktor zunächst mit auf 340 °C vorgeheiztem Phosgen beaufschlagt, bis sich eine konstante Temperatur einstellt. Dann wird die Reaktion gestartet, indem mit der Dosierung von Amin begonnen wird. Das Amin wird mit Hilfe der Förderpumpe durch die mit der Aminvorlage verbundene Rohrleitung in den Verdampfer geleitet, dort verdampft und dann als Dampf über eine Düse in den Reaktor eingespeist. Der erforderliche Massenstrom an Amin wird mit Hilfe eines Regelventils in der Rohrleitung zwischen Förderpumpe und Verdampfer eingestellt. Nach einer Laufzeit von ca. 1 Stunde wird die Reaktion für ca. 15 Minuten unterbrochen, indem die Aminzufuhr, die Energiezufuhr des Heizelements und die Energiezufuhr der Begleitheizung gestoppt werden. Nach Ablauf der 15 Minuten gelingt es problemlos, die Reaktion wieder zu starten, indem in kurzer Abfolge zunächst die Aminzufuhr und dann die Energiezufuhr von Heizelement und Begleitheizung wieder in Betrieb genommen werden. Der Solldurchsatz an Amin kann rasch wieder eingeregelt werden.

### Beispiel 2 (2,4-TDI lange Unterbrechung)

Der Versuch aus Beispiel 1 wird wiederholt. Diesmal wird während der Unterbrechung das Amin mit Hilfe von Stickstoff aus der Rohrleitung zurück in die Aminvorlage gedrückt und die Rohrleitung auf diese Weise entleert. Erst am folgenden Tag wird das Amin in der Aminvorlage zunächst wieder auf 123 °C erwärmt und dabei aufgeschmolzen. Die Reaktion kann wie üblich angefahren werden. Die Aminversorgung durch die Rohrleitung unterliegt keinen störenden Durchflussbeschränkungen.

### Beispiel 3 (PACM)

Der Versuch aus Beispiel 1 wird mit 4,4'-Diaminodicyclohexylmethan als Amin wiederholt. Die Temperatur in der Aminvorlage wird für diesen Versuch auf 90 °C geregelt und der Phosgenstrom auf 360 °C erwärmt. Ansonsten wird verfahren wie in Beispiel 1. Auch bei dieser Ausführung gelingt es nach Unterbrechung problemlos, die Reaktion wieder zu starten, indem in kurzer Abfolge zunächst die Aminzufuhr und dann die Energiezufuhr von Heizelement und Begleitheizung wieder in Betrieb genommen werden. Der Solldurchsatz an Amin kann rasch wieder eingeregelt werden.

## Patentansprüche

1. Verfahren zur Herstellung von Isocyanaten durch Phosgenierung von Aminen mit Phosgen, bei dem das Amin in einer Aminvorlage, die über wenigstens eine Rohrleitung mit einem Reaktor verbunden ist, in flüssiger Form bereitgestellt wird, wobei der Rohrleitung gegebenenfalls als weitere Apparate ein oder mehrere Fördereinrichtungen, Sensoren, Aktoren, Wärmetauscher und/oder Mischeinrichtungen zugeordnet sind, und das Amin aus der Aminvorlage durch die Rohrleitung und gegebenenfalls dieser Rohrleitung zugeordnete Apparate in den Reaktor transferiert und im Reaktor mit einem Überschuss an Phosgen zum Isocyanat umgesetzt wird,
**dadurch gekennzeichnet, dass** die Temperatur von mit dem flüssigen Amin in Kontakt stehenden Oberflächen der Aminvorlage, der Rohrleitung und der gegebenenfalls vorhandenen, der Rohrleitung zugeordneten Apparate, wenigstens 5 K oberhalb der Schmelztemperatur des Amins gehalten wird, wobei mindestens eines der folgenden Merkmale erfüllt ist:
a) Beheizen der Aminvorlage
b) Beheizen des Amins
c) Beheizen der Rohrleitung und/oder gegebenenfalls dieser zugeordneter Apparate
d) Isolieren der Aminvorlage
e) Isolieren der Rohrleitung und/oder gegebenenfalls dieser zugeordneter Apparate.

2. Verfahren gemäß Anspruch 1, **dadurch gekennzeichnet, dass** das Amin eine Schmelztemperatur oberhalb von 5 °C aufweist.

3. Verfahren gemäß einem der Ansprüche 1 oder 2, **dadurch gekennzeichnet, dass** die Phosgenierung kontinuierlich, semikontinuierlich oder diskontinuierlich erfolgt.

4. Verfahren gemäß einem der Ansprüche 1 oder 2, **dadurch gekennzeichnet, dass** die Phosgenierung in der Gasphase oder in flüssiger Phase erfolgt, wobei sie in der Gasphase bevorzugt kontinuierlich oder semikontinuierlich und in flüssiger Phase bevorzugt kontinuierlich, semikontinuierlich oder diskontinuierlich erfolgt.

5. Verfahren gemäß einem der Ansprüche 1 bis 4, **dadurch gekennzeichnet, dass** die Temperatur von mit dem flüssigen Amin in Kontakt stehenden Oberflächen der Aminvorlage, der Rohrleitung und der gegebenenfalls vorhandenen, der Rohrleitung zugeordneten Apparate wenigstens 10 K, bevorzugt wenigstens 20 K und besonders bevorzugt wenigstens 30 K oberhalb der Schmelztemperatur des Amins gehalten wird.

6. Verfahren gemäß einem der Ansprüche 1 bis 5, **dadurch gekennzeichnet, dass** im Falle eines geplanten oder ungeplanten Abfahrens der der Phosgenierung, das Amin zumindest teilweise aus einem oder mehreren aminführenden Anlagenteilen entfernt wird, wobei das entfernte Amin vorzugsweise in einen Sammelbehälter transferiert wird.

7. Verfahren gemäß einem der Ansprüche 1 bis 6, **dadurch gekennzeichnet, dass** die Temperatur der mit dem flüssigen Amin in Kontakt stehenden Oberflächen 180 °C, bevorzugt 150 °C und besonders bevorzugt 120 °C nicht überschreitet.

8. Phosgenierungsanlage zur Durchführung des Verfahrens gemäß den Ansprüchen 1 bis 7, umfassend oder bestehend aus
I) mindestens eine Aminvorlage zur Bereitstellung eines flüssigen Aminstroms,
II) mindestens eine Phosgenstrom-Vorrichtung zur Bereitstellung eines Phosgenstroms,
III) einen Reaktor zur Vermischung der Ströme aus (I) und (II) und Umsetzung des Phosgenstroms mit dem Aminstrom,
IV) wobei von der Aminvorlage mindestens eine Rohrleitung zur Beförderung des zumindest zunächst flüssigen Amins zum Reaktor abgeht,
**dadurch gekennzeichnet, dass** die Phosgenierungsanlage wenigstens ein Temperatursteuerungssystem oder Temperaturregelungssystem umfasst, das eines oder mehrere Heizvorrichtungen zur direkten und/oder indirekten Erwärmung des Amins aufweist.

9. Vorrichtung gemäß Anspruch 8, **dadurch gekennzeichnet, dass** die Phosgenierungsanlage ein Temperaturregelungssystem umfasst, das mindestens einen Temperatursensor zur Messung der Temperatur des Amins in der Aminvorlage und mindestens eine Steuereinheit, die mit dem Temperatursensor und einem oder mehreren Heizvorrichtungen in Verbindung steht, umfasst, wobei die Steuereinheit zur Regelung der Temperatur des flüssigen Amins in der Aminvorlage, bevorzugt durch Steuerung der Leistung der Heizvorrichtungen, eingerichtet ist.

10. Vorrichtung gemäß Anspruch 8 oder 9, **dadurch gekennzeichnet, dass** mindestens eine Heizvorrichtung der Rohrleitung zugeordnet ist, wobei die Heizvorrichtung entlang eines wesentlichen Teils der Rohrleitung verläuft und dazu eingerichtet ist, die Rohrleitung und damit indirekt das flüssige Amin im inneren der Rohrleitung zu erwärmen.

11. Vorrichtung gemäß einem der Ansprüche 8 bis 10, **dadurch gekennzeichnet, dass** ein wesentlicher Teil der Rohrleitung mit einer Isolierung versehen ist.

12. Vorrichtung gemäß einem der Ansprüche 8 bis 11, **dadurch gekennzeichnet, dass** die Phosgenstrom-Vorrichtung einen gasförmigen Phosgenstrom bereitstellt, gegebenenfalls umfassend neben Phosgen einen Inertstoff im Phosgenstrom und die Vorrichtung mindestens eine Aminstrom-Vorrichtung zur Überführung des flüssigen Aminstroms in einen gasförmigen Aminstrom, gegebenenfalls umfassend neben Amin einen Inertstoff im Aminstrom, umfasst und gegebenenfalls mindestens eine Inertstoffstrom-Vorrichtung zur Bereitstellung eines Inertstoffstroms umfasst.

13. Verwendung mindestens einer Heizvorrichtung und mindestens einer Isolierung mit wenigstens einem Temperatursteuerungssystem oder Temperaturregelungssystem, zur Bewahrung des flüssigen Zustands eines Amins in einem Verfahren zur Herstellung von Isocyanaten durch Phosgenierung von Aminen über wenigstens 2 Minuten, bevor das Amin aufgrund von Wärmeverlusten seine Schmelztemperatur unterschreitet und sich verfestigt.

14. Verwendung gemäß Anspruch 13, **dadurch gekennzeichnet, dass** der flüssige Zustand des Amins wenigstens 5 Minuten, bevorzugt wenigstens 15 Minuten und besonders bevorzugt wenigstens 30 Minuten bewahrt wird.

15. Verwendung gemäß Anspruch 13 oder 14, **dadurch gekennzeichnet, dass** die Bewahrung des flüssigen Zustands während einer Produktionsunterbrechung stattfindet.
